# EUROPÄISCHE PATENTANMELDUNG

(11) **EP 2 030 567 A1**
(43) Veröffentlichungstag der Anmeldung: **04.03.2009**
(21) Anmeldenummer: 07017059.2
(22) Anmeldetag: 31.08.2007
(51) Int. Cl.: A61B 5/15

(54) **Analysegerät zur Bestimmung eines Analyten in einer Körperflüssigkeit, Magazin für ein Analysegerät und Verfahren zur Erzeugung einer Wunde und zum Untersuchen einer austretenden Körperflüssigkeit**

(71) Anmelder: Roche Diagnostics GmbH, 68305 Mannheim (DE); F.HOFFMANN-LA ROCHE AG, 4070 Basel (CH)
(72) Erfinder: Frey, Stephan-Michael, 64347 Griesheim (DE); List, Hans, 64754 Hesseneck-Kailbach (DE); Flügge, Kai, 52070 Aachen (DE); Zimmer, Volker, 67229 Laumersheim (DE); Hörauf, Christian, 68723 Oftersheim (DE); Rittinghaus, Andrea, 69239 Neckarsteinach (DE)
(74) Vertreter: Petirsch, Markus

(57) **Zusammenfassung**

Analysesystem zur Bestimmung eines Analyten in einer Körperflüssigkeit, umfassend ein wiederverwendbares Analysegerät (2) und ein Magazin (3) mit einer Mehrzahl von Stechelementen (21) und mit einer Mehrzahl von Analyseelementen (26), die eine Probenkontaktzone 27 und ein Reagenzsystem mit wenigstens einer eine Reagenz enthaltene Komponente umfassen, dessen Reaktion mit einer Körperflüssigkeit eine messbare Änderung einer Messgröße erzeugt, wobei das Analysegerät (2) umfasst einen Antrieb (5), durch den die Stechbewegung des Stechelements (21) angetrieben wird, um es auf einem Bewegungsweg während einer Vortriebsphase in Einstichrichtung und nach Erreichen eines Umkehrpunktes der Stechbewegung während einer Rückführungsphase entgegen der Einstichrichtung zu bewegen, eine Kopplungseinheit (6) mit einem Kopplungselement (7), die dazu ausgebildet ist, ein Stechelement (21) mit dem Antrieb (5) zu koppeln, eine Mess- und Auswerteeinheit (8) zur Messung einer für die Bestimmung eines Analyten charakteristischen Messgröße und zur Ermittlung eines gewünschten Analyseergebnisses und eine Halterung (10) zur Aufnahme des Magazins (3), das Magazin (3) umfasst eine Mehrzahl von nebeneinander angeordneten langgestreckten Kammern (16), die durch zwei in Längsrichtung der Kammern (16) verlaufende Seitenwände (17) begrenzt sind und deren Höhe und Länge größer ist als ihre durch den Abstand zweier benachbarter Seitenwände (17) definierte Breite, wobei die Kammern (16) von den Seitenwänden (17) begrenzte Hauptseiten sowie je zwei sich zwischen den Seitenwänden (14) erstreckende lange und kurze Schmalseiten (19a,19b,20a,20b) aufweisen, wenigstens eine Kammer (16) ein Stechelement (21) mit einer Spitze (29) enthält, das eine Kopplungsstruktur (39) aufweist, um das Stechelement (21) in einer Ausgangsposition an das Kopplungselement (7) der Kopplungseinheit (6) zu koppeln, und das auf dem Bewegungsweg der Stechbewegung in Einstichrichtung bewegbar ist, die Kammern (16) an der in Einstichrichtung vorderen kurzen Schmalseite (20a) eine Austrittsöffnung (20c) aufweisen durch die ein auf dem Bewegungsweg bewegtes Stechelement (21) wenigstens teilweise aus der Kammer (16) heraustreten kann, die Analyseelemente (26) stationär in Bezug auf das Magazin (3) angeordnet sind, das Stechelement (21) eine Probenübergabezone (32) und einen Kapillarkanal (34) mit wenigstens einem Probeneinlass (35) und einem Probenauslass (36) hat, der Bewegungsweg des Stechelements (21) eine Übergabeposition aufweist, in der das Stechelement (21) relativ zu dem Analyseelement (26) derart angeordnet ist, dass die Probenübergabezone (32) des Stechelements (21) der Probenkontaktzone (27) des Analyseelements (26) so benachbart ist, dass eine Übertragung einer Körperflüssigkeit aus dem Kapillarkanal (34) auf die Probenkontaktzone (27) stattfinden kann.

## Beschreibung

Die vorliegende Erfindung betrifft ein Verfahren zur Bestimmung eines Analyten in einer Körperflüssigkeit umfassend ein wiederverwendbares Analysegerät und ein Magazin mit einer Mehrzahl von Stechelementen und einer Mehrzahl von Analyseelementen. Die Analyseelemente umfassen eine Probenkontaktzone und ein Reagenzsystem mit wenigstens einer eine Reagenz enthaltende Komponente, dessen Reaktion mit einer Körperflüssigkeit eine messbare Änderung einer Messgröße erzeugt. Das Analysegerät umfasst einen Antrieb, durch den die Stechbewegung des Stechelements angetrieben wird, um es auf einem Bewegungsweg während einer Vortriebsphase in Einstichrichtung und nach Erreichen eines Umkehrpunktes der Stechbewegung während einer Rückführungsphase entgegen der Einstichrichtung zu bewegen. Das Analysegerät umfasst weiter eine Kopplungseinheit mit einem Kopplungselement, die dazu ausgebildet ist, ein Stechelement mit dem Antrieb zu koppeln, und eine Mess- und Auswerteeinheit zur Messung einer für die Bestimmung eines Analyten charakteristischen Messgröße und zur Ermittlung eines gewünschten Analyseergebnisses. Weiterhin umfasst das Analysegerät eine Halterung zur Aufnahme des Magazins.

Die Erfindung betrifft auch ein Magazin, das eine Mehrzahl von Stechelementen und eine Mehrzahl von Analyseelementen aufweist. Ein Verfahren zum Erzeugen einer Wunde in einem Körperteil und zur Untersuchung einer aus der Wunde austretenden Körperflüssigkeit ist ebenfalls Bestandteil der Erfindung. Das Verfahren wird mittels eines Analysesystems ausgeführt, welches ein wiederverwendbares. Analysegerät und ein Magazin mit einer Mehrzahl von Stechelementen und mit einer Mehrzahl von Analyseelementen umfasst.

Die Bestimmung eines Analyten aus einer Körperflüssigkeit, insbesondere Blut, ist auf vielen Gebieten der medizinischen Analyse und Behandlung wichtig. Um einen Analyten, beispielsweise den Glucosegehalt, in einer Körperflüssigkeit für medizinische und diagnostische Zwecke zu bestimmen, werden geringe Mengen der Körperflüssigkeit aus einem Körperteil, vorzugsweise aus der Fingerbeere entnommen. Die zum Einsatz kommenden Stechgeräte mit einer Lanzette sind so konstruiert, dass sie nicht nur von medizinischem Personal, sondern auch von Laien verwendet werden können.

Der Vorgang zur Bestimmung des Analyten besteht aus einem Stechvorgang mit einem separaten Stechgerät und aus einem Analysevorgang mit einem zweiten Gerät, dem Analysegerät. Der Benutzer muss zwei unabhängig voneinander arbeitende Geräte verwenden, eines zur Erzeugung der Wunde und ein zweites zur Bestimmung des Analyten. Dies ist für den Benutzer recht unkomfortabel und umständlich.

Deshalb wurden kombinierte Systeme entwickelt, die die beiden bisherigen Geräte in einem Gerät zu vereinen und beide Schritte in einem Gerät ausführen können. Die im Stand der Technik bekannten und auf dem Markt erhältlichen Geräte sind jedoch in ihrer Baugröße recht groß.

Bekannt sind auch integrierte Analysesysteme, die ein integriertes Probengewinnungs- und Analyseelement aufweisen. Derartige Systeme lassen sich sehr gut automatisieren, so dass mit Ihnen ein "one-steptreatment" möglich ist. Der Benutzer muss das System nur einmal ansetzen und kann das Analyseergebnis - ohne weitere Handlungsschritte - ablesen. Die Systeme sind in ihrem mechanischen Aufbau jedoch sehr kompliziert und dementsprechend teuer in der Herstellung.

Ein Problem bei den automatisch arbeitenden integrierten Systemen ist der Bluttransfer aus der Wunde zu einer Analyseeinheit, um den Analyten zu bestimmen. Um ein kompaktes System zu entwickeln, müssen sehr kleine Mengen an Blut transportiert und weiterverarbeitet werden. Weitere Probleme betreffen die Bewegungs- und Ankopplungsmechanik sowie die Kontaminierung der Lanzetten durch Bestandteile der Analyseelemente, da bei den integrierten Probengewinnungs- und Analyseelementen die Lanzetten und Analyseelemente auf engstem Raum benachbart sind.

Beispiele für die Bemühungen der Fachwelt, integrierte Analysesysteme zu verbessern, sind in den folgenden Druckschriften beschrieben:
1. WO 2006/092281
2. US 6,607,658 B1
3. WO 2006/027101 A1
4. EP 1287785 A1
5. EP 1658897 A1
6. US 2003/0212345 A1

Es besteht die Aufgabe, die bekannten integrierten Geräte zu verbessern. Insbesondere existiert ein großes Bestreben, preiswerte integrierte Geräte zu schaffen, die eine kompakte Baugröße aufweisen.

Gelöst wird das zugrundeliegende Problem durch ein Analysesystem mit den Merkmalen des Anspruchs 1, ein Magazin mit den Merkmalen des Anspruchs 4 und des Anspruchs 16 sowie durch ein Verfahren mit den Merkmalen des Anspruchs 19.

Die auf die unabhängigen Ansprüche rückbezogenen Unteransprüche definieren bevorzugte Weiterbildungen der Gegenstände der unabhängigen Ansprüche.

Erfindungsgemäß umfasst das Analysesystem zur Bestimmung eines Analyten in einer Körperflüssigkeit ein wiederverwendbares Analysegerät und ein Magazin mit einer Mehrzahl von Stechelementen und mit einer Mehrzahl von Analyseelementen. Das Analysegerät weist einen Antrieb, eine Kopplungseinheit mit einem Kopplungselement, eine Mess- und Auswerteeinheit und eine Halterung zur Aufnahme des Magazins auf.

Der Antrieb treibt die Stechbewegung des Stechelementes an, um es auf seinem Bewegungsweg während einer Vortriebsphase in Einstichrichtung und nach Erreichen eines Umkehrpunktes der Stechbewegung während einer Rückführungsphase entgegen der Einstichrichtung zu bewegen. Die Kopplungseinheit ist derart ausgebildet, dass ein Stechelement mit dem Antrieb gekoppelt werden kann. Die Mess- und Auswerteeinheit ist geeignet zur Messung einer für die Bestimmung eines Analyten charakteristischen Messgröße und zur Ermittlung eines gewünschten Analyseergebnisses.

Das Magazin umfasst erfindungsgemäß eine Mehrzahl von nebeneinander angeordneten Kammern. Wenigstens eine der Kammern des Magazins enthält ein Stechelement mit einer Kopplungsstruktur derart, dass das Stechelement in einer Ausgangsposition an das Kopplungselement der Kopplungseinheit ankoppelbar ist. Das in der Kammer enthaltene Stechelement kann auf dem Bewegungsweg der Stechbewegung in Einstichrichtung bewegt werden. In Einstichrichtung weisen die Kammern je eine Austrittsöffnung auf, durch die das Stechelement wenigstens teilweise aus der Kammer heraustreten kann. Grundsätzlich kann das Stechelement auch vollständig aus der Kammer heraustreten oder aus ihr entnommen werden.

Die Analyseelemente des Magazins sind stationär in Bezug auf das Magazin angeordnet. Im Sinne der vorliegenden Erfindung wird der Begriff "stationär" so verstanden, dass die Analyseelemente wenigstens während des gesamten Stech- und Analysevorgangs eine unlösbare Verbindung mit dem Magazin aufweisen. Die Analyseelemente sind somit dauerhaft an dem Magazin angeordnet. Die fixierten Analyseelemente sind demnach ortsfest relativ zu dem Magazin, so dass eine Bewegung des Magazins auch eine Bewegung der Analyseelement zur Folge hat. Die Analyseelemente sind derart ausgebildet, dass sie eine Probenkontaktzone und ein Reagenzsystem mit wenigstens einer Komponente umfassen, die eine Reagenz enthält. Die Reaktion des Reagenzsystems mit einer Körperflüssigkeit erzeugt eine messbare Änderung einer Messgröße, die Grundlage zur Ermittlung des gesuchten Analyseergebnisses ist.

Das Stechelement des Analysesystems weist eine Probenübergabezone und einen Kapillarkanal auf, der wenigstens einen Probeneinlass und einen Probenauslass umfasst. Der Bewegungsweg des Stechelements umfasst eine Übergabeposition, in der das Stechelement in Bezug auf das Analyseelement derart angeordnet ist, dass eine Übertragung einer Körperflüssigkeit vom Stechelement auf das Analyseelement stattfinden kann. Die Probenübergabezone des Stechelements ist der Probenkontaktzone des Analyseelements derart benachbart, dass die Körperflüssigkeit aus dem Kapillarkanal auf die Probenkontaktzone übertragen wird. Das Stechelement und das Analyseelement können auch in direktem Kontakt stehen. Die Bestimmung des gesuchten Analyten kann entweder durch eine optische Analyse oder durch elektrochemische Messung stattfinden.

Ein Vorteil des erfindungsgemäßen Analysesystems ist der sehr einfache Bewegungsablauf, der durch eine einfache Mechanik realisiert werden kann. Da das Analyseelement relativ zu dem Magazin permanent fixiert ist, wird die Übergabeposition durch die Position des Analyseelements festgelegt. Es muss ausschließlich das Stechelement bewegt werden. Hierzu ist nur ein Antrieb erforderlich; auf zusätzliche Antriebe kann verzichtet werden. Es ist somit ohne großen technischen Aufwand möglich, das Stechelement und das Analyseelement sehr exakt zueinander zu positionieren, damit eine Flüssigkeitsübertragung stattfinden kann. Die Übergabeposition des Stechelements, die Teil seines Bewegungsweges ist, wird während der Rückführungsphase der Stechbewegung dadurch erreicht, dass die Rückführbewegung fortgeführt wird. Die Einstichbewegung und die Bewegung in die Übergabeposition liegen somit auf einer Achse des Systems.

Das Stechelement wird folglich unabhängig von dem Analyseelement bewegt. Es findet lediglich eine Bewegung in und entgegen der Einstichrichtung statt, um sowohl den Einstich auszuführen, als auch die Übergabeposition des Stechelements zu erreichen, in dem eine Körperflüssigkeit (z.B. Blut) vom Stechelement zu dem Analyseelement übertragen wird. Eine weitere Bewegung, beispielsweise quer zur Einstichrichtung, um die Übergabeposition zu erreichen, ist nicht notwendig.

Das erfindungsgemäße Analysesystem kann folglich sehr klein aufgebaut werden, so dass es eine kompakte Bauform aufweist und recht leicht ist. Darüberhinaus ist der mechanische Antrieb des Analysesystems äußerst robust, da wenige mechanische Komponenten benötigt werden. Das System lässt sich besonders gut automatisieren, so dass der Benutzer das System nur einmal an seinen Finger andrücken muss und kurze Zeit später das gewünschte Analyseergebnis an dem Gerät ablesen kann. Eine Vorgehensweise, die als "One-Stop-Handlung" bezeichnet wird.

Eine besonders bevorzugte Ausführungsform der Erfindung umfasst ein Magazin mit einer Mehrzahl von langgestreckten, nebeneinander angeordneten Kammern, die durch zwei in Längsrichtung der Kammern verlaufende Seitenwände begrenzt sind. Die Höhe und Länge der Kammern ist größer als die durch den Abstand der beiden benachbarten Seitenwände definierte Breite. Die langgestreckten Kammern haben zwei von den Seitenwänden begrenzte Hauptseiten sowie zwei lange und zwei kurze Schmalseiten, die sich zwischen den Seitenwänden erstrekken. Die Austrittsöffnung der Kammern liegt an der in Einstichrichtung vorderen kurzen Schmalseite.

In einer bevorzugten Ausführungsform umfasst die Mess- und Auswerteeinheit eine optische Messeinrichtung, um eine optisch messbare Messgröße zu messen, die für die Bestimmung des Analyten charakteristisch ist. Neben Messverfahren, die auf Luminiszenz- oder Fluoreszenz-Messungen beruhen, wird besonders ein auf einer diffusen Reflexion beruhendes Messverfahren bevorzugt. Mit einer optischen Messeinrichtung lassen sich sehr gut und einfach Änderungen im Reagenzsystem der Analyseelemente detektieren. Die optische Messeinrichtung ermöglicht eine kontaktlose Messung. Ein weiterer Vorteil besteht darin, dass mit der Optik auch erkannt werden kann, ob ein Stechelement in einer Kammer des Magazins enthalten ist und in welcher Position es sich befindet. Beispielsweise kann aus der detektierten Position des Stechelements festgestellt werden, ob das Stechelement unbenutzt ist oder ob es bereits für einen Einstich verwendet wurde. Beim Erkennen eines bereits benutzten Stechelements kann ein erneutes Ankoppeln der Kopplungseinheit durch das System automatisch verhindert werden.

Das erfindungsgemäße Magazin für ein Analysegerät, das vorzugsweise Bestandteil des erfindungsgemäßen Analysesystems ist, weist den Vorteil auf, dass die Analyseelemente dauerhaft ortsfest in Relation zu dem Magazin angeordnet sind. Insbesondere bei einer optischen Messung einer für die Bestimmung des Analyten charakteristischen Messgröße des Reagenzsystems des Analyseelements ist eine einfache und genaue Positionierung des Analyseelements in Bezug zu der Messund Auswerteeinheit möglich. Die Messung wird hierdurch sehr zuverlässig. Beispielsweise kann das Analyseelement im in Einstichrichtung hinteren Teil der Kammern angeordnet sein. Zur Übertragung einer Flüssigkeit vom Stechelement zu dem Analyseelement wird das Stechelement während seiner Rückführungsphase beispielsweise soweit bewegt, bis das Stechelement vollständig in der Kammer enthalten ist. Das Analyseelement kann zum Beispiel an einer der Seitenwände an einer der langen Schmalseiten der Kammer angeordnet sein.

Bevorzugt weisen die Kammern des Magazins an der in Einstichrichtung hinteren Seite eine Analyseöffnung auf. Die Analyseöffnung liegt somit den Austrittsöffnungen für das Stechelement gegenüber. Durch die Analyseöffnungen kann die Messung der für den Analyten charakteristischen Messgröße stattfinden. Vorzugsweise hat das Magazin an der in Einstichrichtung hinteren Seite einen Deckel, der die Analyseöffnungen der Kammern (wasserdampfdicht) verschließt. Damit eine optische Messung möglich ist, ist der Deckel bevorzugt aus einem durchsichtigen bzw. transparenten Material, wie beispielsweise Kunststoff. Das Analyseelement kann auch derart in den Deckel integriert sein, dass eine Detektionsfläche des Reagenzsystems, an der die optische Messeinrichtung eine Reaktion mit der Probenflüssigkeit detektieren kann, auf der an den Kammern abgewandten Seite des Deckels angeordnet ist. Die Detektionsfläche liegt in diesem Fall der Probenkontaktfläche gegenüber. Das Reaktionssystem ist folglich mehrschichtig aufgebaut, wobei wenigstens eine Schicht auf jeder Seite des Deckels angeordnet ist. In diesem Fall muss der Deckel selbst nicht transparent sein.

Die Verwendung eines Deckels an der hinteren Seite des Magazins hat den Vorteil, dass mit einem Bauteil sämtliche Austrittsöffnungen der Kammern verschlossen werden können.

Das erfindungsgemäße Magazin hat darüber hinaus den Vorteil, dass die Kammern des Magazins benachbart zueinander angeordnet sind, so dass auf engstem Raum eine Vielzahl von Kammern in dem Magazin realisiert werden kann. Zehn, fünfundzwanzig oder bis zu fünfzig Stechelemente lassen sich in recht kleinen Magazinen anordnen.

Das erfindungsgemäße Stechelement hat eine Probenübergabezone und einen Kapillarkanal mit wenigstens einem Probeneinlass und einem Probenauslass. Der Kapillarkanal ist bevorzugt eine offene Kapillarstruktur. Vorteilhaft ist eine Kapillare, die einseitig offen ist und z.B. einen rinnenartigen, halbrunden oder gebogenen Querschnitt hat. Der Kapillarkanal kann auch beidseitig offen sein. Das Stechelement weist eine Kopplungsstruktur auf, an die ein Kopplungselement einer Kopplungseinheit ankoppelbar ist. Das Stechelement hat zwei Schenkel. Der eine Schenkel ist ein Übergabeschenkel, der sich quer zur Einstichrichtung erstreckt und an dem die Probenübergabezone angeordnet ist. Der andere Schenkel ist ein Stechschenkel, der sich in Einstichrichtung erstreckt. Er umfasst die Spitze des Stechelements und den Probeneinlass des Kapillarkanals. Eine in dem Kapillarkanal enthaltene Flüssigkeit wird in die Probenübergabezone transferiert.

Bevorzugt wird die Probenübergabezone von dem Probenauslass des Kapillarkanals gebildet und hat bevorzugt einen rinnenartigen oder halbrunden Querschnitt. In diesem Fall erstreckt sich der Kapillarkanal vom Einstechschenkel in den Übergabeschenkel derart, dass der Kapillarkanal gebogen ist und dabei eine Biegung um 90° aufweist. Der Flüssigkeitstransfer innerhalb des Kapillarkanals ist jedoch auch mit einer 90°-Biegung gewährleistet, so dass die Flüssigkeit von dem Stechschenkel in den quer angeordneten Übergabeschenkel transportiert wird.

Bevorzugterweise ist der Übergabeschenkel des Stechelements Bestandteil der Kopplungsstruktur. Er hat dann eine Doppelfunktion. Beispielsweise kann er einen Teil einer klammerartigen Kopplungsstruktur bilden, die vorzugsweise so ausgebildet ist, dass ein Kopplungselement formschlüssig ankoppeln kann.

Erfindungsgemäß erfolgt das Erzeugen einer Wunde in einem Körperteil und das Untersuchen einer aus der Wunde austretenden Körperflüssigkeit mittels eines Analysesystems, das ein wiederverwendbares Analysegerät und ein Magazin mit einer Mehrzahl von Stechelementen und einer Mehrzahl von Analyseelementen umfasst.

Zur Durchführung des Verfahrens wird ein System mit einem Antrieb, einer Kopplungseinheit mit einem Kopplungselement, einer Mess- und Auswerteeinheit und einer Halterung zur Aufnahme des Magazins verwendet. Das Magazin hat eine Mehrzahl von nebeneinander angeordneten Kammern, die ein Stechelement mit einer Kopplungsstruktur enthalten. Die Kammern sind derart zugänglich, dass das Kopplungselement der Kopplungseinheit formschlüssig an die Kopplungsstruktur des Stechelements ankoppelbar ist.

Zuerst wird ein Kopplungselement einer Kopplungseinheit an eine korrespondierende Kopplungsstruktur des Stechelements formschlüssig angekoppelt, wobei das (unbenutzte) Stechelement in einer Ausgangsposition in einer Kammer des Magazins positioniert ist. In einem weiteren Schritt wird das Stechelement (unabhängig vom Analyseelement) auf seinem Bewegungsweg in Einstichrichtung bis zum Erreichen eines Umkehrpunktes bewegt und eine Einstichwunde in der Haut eines vor dem Stechelement angeordneten Körperteils erzeugt. Die austretende Körperflüssigkeit wird durch einen Probeneinlass in einen Kapillarkanal des Stechelements übertragen. Ein weiterer Schritt des Verfahrens sieht vor, dass das Stechelement entgegen der Einstichrichtung in eine Übergabeposition bewegt wird, in der das Stechelement in der Kammer des Magazins so positioniert ist, dass seine Übergabezone zu der Probenkontaktzone des Analyseelements des Magazins benachbart ist und eine Übertragung der Körperflüssigkeit aus dem Kapillarkanal des Stechelements auf die Probenkontaktzone stattfindet. In einem nächsten Schritt wird eine Messgröße gemessen, die für die Bestimmung eines Analyten charakteristisch ist. Die "Vermessung" (d.h. das Messen der Messgröße und das Bestimmen des Analyten) des Analyseelements erfolgt, wenn das Stechelement wieder in der Kammer angeordnet ist. Der Bewegungsablauf des Stechelements ist dann abgeschlossen. Das Stechelement befindet sich im Stillstand. Bevorzugt ist das Stechelement zum Zeitpunkt der Messung bereits remagaziniert. Es ist in einer Endposition gelagert, in der es nach Gebrauch fixiert ist. Die Spitze des Stechelementes ist dabei bevorzugt ebenfalls in der Kammer enthalten.

In einer bevorzugten Ausführungsform weisen die Kammern des Magazins an der in Einstichrichtung hinteren kurzen Schmalseite Analyseöffnungen auf, die den Austrittsöffnungen der Kammern gegenüber liegen. Die Analyseöffnungen der Kammern werden durch einen Deckel des Magazins verschlossen, an dem die Analyseelemente derart angeordnet sind, dass die Probenkontaktzone des Analyseelements mit der Analyseöffnung der Kammer fluchtet.

In einer bevorzugten Ausführungsform des Verfahrens wird das Stechelement mit quer zur Einstichrichtung angeordnetem Übergabeschenkel während der Rückführungsphase so weit entgegen der Einstichrichtung bewegt, dass der Übergabeschenkel mit seiner Probenübergabezone dem Deckel des Magazins benachbart ist oder an ihm anliegt. In dieser Übergabeposition ist der Probentransfer vom Stechelement zum Analyseelement möglich. Bevorzugt wird das Stechelement in der Übergabeposition gehalten, z.B. durch kraft- oder reibschlüssiges Halten in der Kammer. Die Verwendung eines Systems mit am Deckel angeordneten Analyseelementen und Stechelementen, die einen Übergabeschenkel aufweisen, hat den Vorteil, dass sowohl zur Ausführung des Einstichs, als auch zum Erreichen der Übergabeposition lediglich eine Bewegung des Stechelements in und entgegen der Einstichrichtung notwendig ist. Weitere Bewegungen, beispielsweise quer zur Einstichrichtung, werden nicht ausgeführt.

Die Erfindung wird nachfolgend anhand von in den Figuren dargestellten bevorzugten Ausführungsformen näher erläutert. Die darin dargestellten Besonderheiten können einzeln oder in Kombination verwendet werden, um bevorzugte Ausgestaltungen der Erfindung zu schaffen. Die beschriebenen Ausführungsformen stellen keine Einschränkung der Allgemeinheit des in den Ansprüchen definierten Gegenstandes dar. Es zeigen:
- Fig. 1: ein Prinzipschaltbild eines Analysesystems;
- Fig. 2: Detailansichten eines Magazins aus Figur 1;
- Fig. 3a,b: Detailansichten des Magazins aus Figur 2:
- Fig. 4: ein Ausschnitt des Magazins aus Figur 2;
- Fig. 5a bis c: ein erfindungsgemäßes Stechelement in perspekti- vischer Ansicht;
- Fig. 6: einen Schnitt durch das Magazin aus Figur 2 mit einem Stechelement.

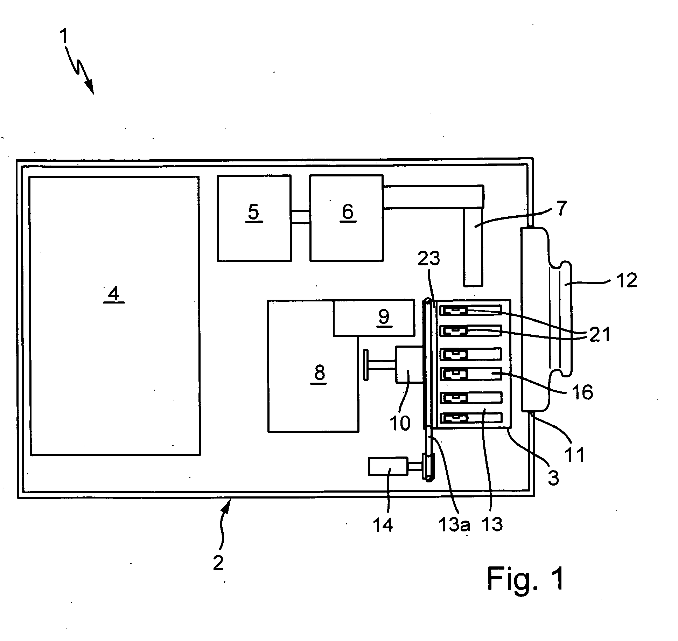

Figur 1 zeigt ein erfindungsgemäßes Analysesystem 1, das ein Analysegerät 2 und ein Magazin 3 umfasst, das in das Gerät 2 eingesetzt ist.

Das Analysegerät 2 weist eine Stromversorgung 4, einen Antrieb 5, eine Kopplungseinheit 6 und eine Mess- und Auswerteeinheit 8, die eine optische Messeinrichtung 9 einschließt. Die Kopplungseinheit 6 hat ein Kopplungselement 7, das an ein in dem Magazin 3 gelagertes Stechelement 21 so ankoppeln kann, dass das Stechelement 21 von dem Antrieb 5 auf seinem Einstichweg bewegt wird.

Das Analysegerät 2 hat eine Halterung 10, um das Magazin 3 hinter einer Gehäuseöffnung 11 zu halten. Die Gehäuseöffnung 11 ist als Fingerkonus 12 ausgebildet, an dem ein Finger zum Erzeugen einer Wunde angelegt wird.

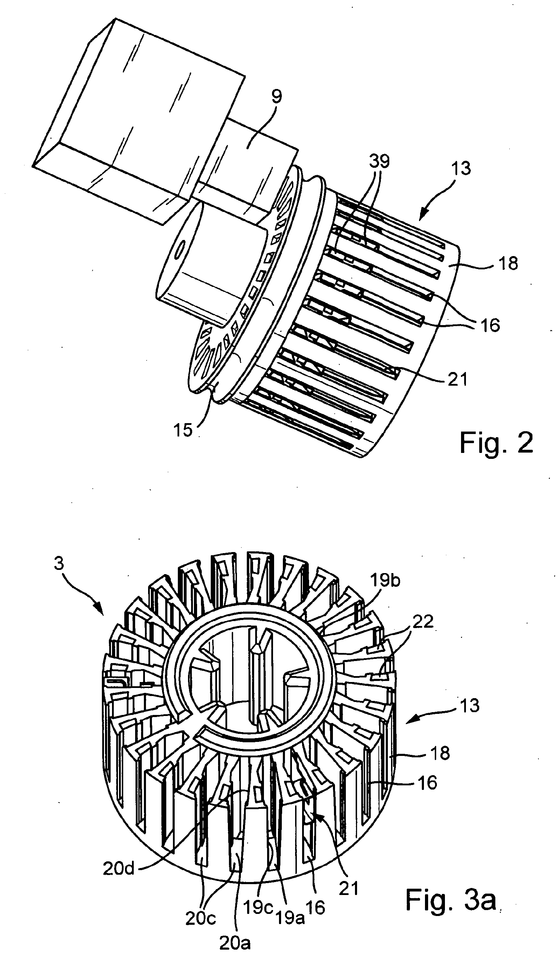

Bevorzugterweise ist das Magazin 3 als Trommelmagazin 13 mit einer Magazintrommel 18 ausgebildet, wie es in den Figuren 1 bis 4 zu erkennen ist. Die Magazintrommel 18 hat eine Mehrzahl von benachbarten Kammern 16, die sich in axialer Richtung des Trommelmagazins 13 erstrecken. Die langgestreckten Kammern 16 enthalten jeweils ein Stechelement 21. Das Trommelmagazin 13 wird von einem Magazinantrieb 14 bewegt, der über einen Riemen mit einer Magazinriemenscheibe 15 gekoppelt ist und das Trommelmagazin 13 in eine gewünschte vorgegebene Position drehen kann. Das Trommelmagazin 13 hat den Vorteil, dass viele Kammern 16 auf engem Raum angeordnet sind und sich eine Kammer 16 einfach positionieren lässt, Figur 2.

Die optische Messeinrichtung 9 ist derart hinter dem Trommelmagazin 13 und der Magazinriemenscheibe 15 angeordnet, dass die Optik der optischen Messeinrichtung 9 auf die in Einstichrichtung hinten liegende Seite der Kammer gerichtet ist, die mit dem Zentrum des (vor dem Magazin 3 angeordnetem) Fingerkonus 12 fluchtet. Die Magazinriemenscheibe 15 weist dazu mehrere Öffnungen auf, die mit der Rückseite der Kammern 16 des Trommelmagazins 13 fluchten, wie aus Figur 2a zu erkennen. Die Ortsangabe hinter dem Trommelmagazin 13 bezieht sich auf die Einstichrichtung.

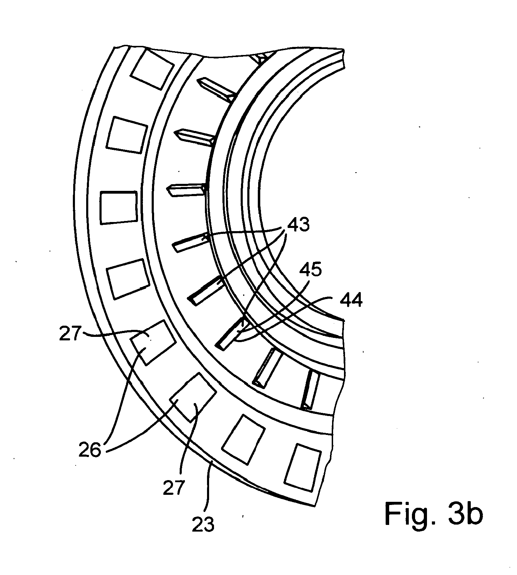

Die Figuren 3a und b zeigen das Trommelmagazin 13 und den zugehörigen Deckel 23. Die langgestreckten, nebeneinander angeordneten Kammern 16 erstrecken sich in Axialrichtung des Trommelmagazins 13. Jede den Kammern 16 hat zwei Seitenwände 17, die zwei Hauptseiten begrenzen, sowie zwei lange Schmalseiten 19a,b und zwei kurze Schmalseiten 20a,b.

Die radial außen angeordnete lange Schmalseite 19a weist bevorzugterweise einen Schlitz 19c auf, durch den die Kammern 16 zugänglich sind. Die radial innen liegende Schmalseite 19b weist vorteilhafterweise eine Führungsnut 19d auf, in der ein in einer Kammer 16 gelagertes Stechelement 21 während der Einstichbewegung geführt werden kann.

Die Kammern 16 enthalten ein Trockenmittel, das als Trockenmittelstab 22 angeordnet ist. Der Trockenmittelstab 22 erstreckt sich ebenfalls in axialer Richtung und ist in einer der Seitenwände 17 in einer dafür vorgesehenen Ausnahme formschlüssig integriert.

Die vordere kurze Schmalseite 20a hat eine Austrittsöffnung 20c, durch die das Stechelement 21 auf seinem Bewegungsweg aus dem Trommelmagazin 13 heraustreten kann. Die hintere kurze Schmalseite 20b, die in Figur 3a sichtbar ist, ist offen und weist bevorzugterweise eine Analyseöffnung 20d auf, die der Austrittsöffnung 20c gegenüber liegt.

Das hintere Ende der Magazintrommel 18 des Trommelmagazins 13 ist bevorzugt mit dem Deckel 23 derart abgedeckt, dass die Analyseöffnungen 20d der Kammern 16 von dem Deckel 23 verschlossen werden. Dies hat den Vorteil, dass mit dem Deckel 23 die Analyseöffnungen 20d aller Kammern 16 steril und wasserdampfdicht verschlossen werden können.

In einer bevorzugten Ausführungsform sind die Analyseelemente 26 derart am Deckel 23 positioniert, dass eine Probenkontaktzone 27 des Analyseelements 26 mit der Analyseöffnung 20d der Kammer 16 fluchtet. Die Analyseelemente 26 sind in Form eines Ringes an dem Deckel 23 angeordnet. Der Deckel 23 weist bevorzugt Aufnahmehalterungen 43 auf, die ebenfalls auch ringförmig am Deckel angeordnet sind und die gegenüber den Analyseelementen 26 radial nach innen versetzt sind. Die Aufnahmehalterungen 43 sind bevorzugt als Schlitz 44 mit Klemmlaschen 45 ausgebildet, in die ein korrespondierendes Halteelement 37 des Stechelements 21 kraftschlüssig eingeklemmt werden kann, um das Stechelement 21 in seiner Endposition zu halten.

Die an den langen Schmalseiten 19a offenen Kammern 16 werden bevorzugt mit einer Folie 24 abgedeckt, die sich über den gesamten Umfang der Magazintrommel 18 erstreckt. Die Austrittsöffnungen 20c der Kammern 16 sind bevorzugt ebenfalls mit einer Folie 25 wasserdampfdicht verschlossen, die vorzugsweise alle Austrittsöffnungen 20c abdeckt. Die Folien 24,25 sind wasserdampfdichte Folien, z.B. aus Kunststoff oder ähnlichem Material. Somit entstehen bevorzugt vollständig geschlossene Kammern 16. Der Innenraum der Kammern 16 ist damit hermetisch gegenüber der Umwelt versiegelt und kann steril gehalten werden, so dass das Stechelement 21 ebenfalls steril bleibt. Der Trockenmittelstab 22 stellt sicher, dass die Kammern 16 innen trocken und somit die Analyseelemente 26 des Magazins 3 trocken gehalten werden. Die an dem Deckel 23 des Magazins 3 angeordneten Analyseelemente 26 werden durch den Trockenmittelstab 22 ebenfalls trocken gehalten, da die Kammern 16 zum Deckel 23 offen sind.

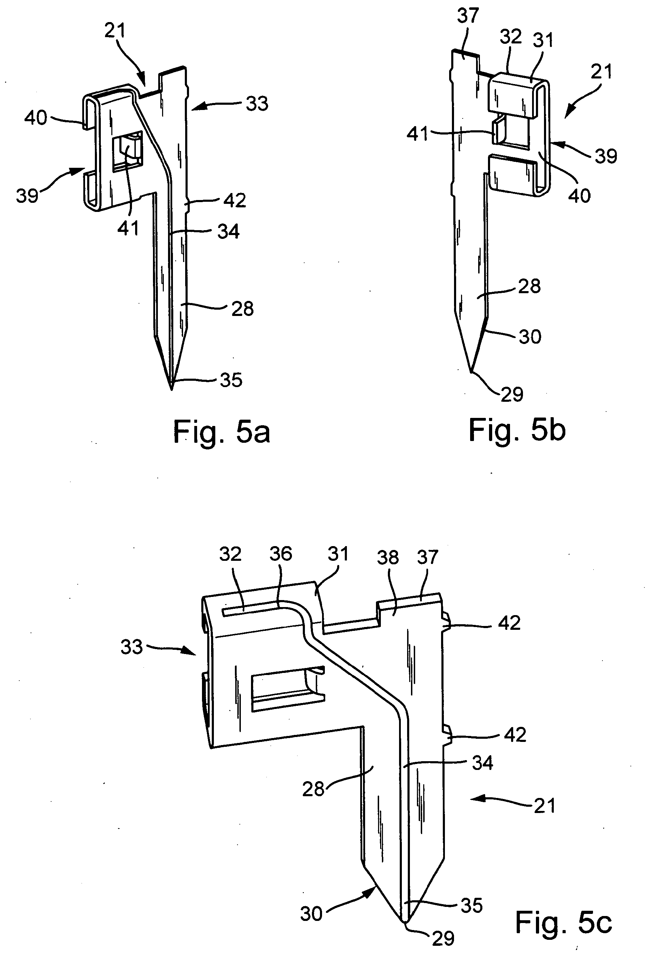

In den Figuren 5a bis 5c ist ein Stechelement 21 in unterschiedlichen perspektivischen Ansichten dargestellt. Das Stechelement 21 weist erfindungsgemäß zwei Schenkel auf, einen Stechschenkel 28, der sich in Einstichrichtung erstreckt und an seinem vorderen Ende 30 eine Spitze 29 aufweist, und einen Übergabeschenkel 31, an dem eine Probenübergabezone 32 angeordnet ist. Bevorzugt erstreckt sich der Übergabeschenkel 31, wie in den Figuren 5 gezeigt, quer zur Einstichrichtung und ist am hinteren Ende 33 des Stechelement 21 angeordnet.

Auf seiner Vorderseite weist das Stechelement 21 einen Kapillarkanal 34 auf, der sich entlang des Stechschenkels 28 in Einstichrichtung erstreckt. Der Kapillarkanal 34 kann beispielsweise einseitig offen sein und eine Art Rinne bildet. Ein Probeneinlass 35 des Kapillarkanals ist am vorderen Ende 30 des Stechschenkels 28 angeordnet. Ein Probenauslass 36 liegt bevorzugt am hinteren Ende 33 des Stechelements 21. Eine in dem Kapillarkanal 34 enthaltene Flüssigkeit kann durch den Probenauslass 36 aus dem Kapillarkanal 34 austreten, bevorzugt auf die Probenübergabezone 32. In einer bevorzugten Ausführungsform wird der Probenauslass 36 von der Probenübergabezone 32 gebildet. Der hier gezeigte Kapillarkanal 34 ist gebogen und erstreckt sich von dem Stechschenkel 28 auf den Übergabeschenkel 31.

Am hinteren Ende 33 weist das Stechelement 21 bevorzugt das Halteelement 37 auf, das im gezeigten Beispiel als Haltezunge 38 ausgebildet ist und sich entgegen der Einstichrichtung erstreckt. Die Haltezunge 38 korrespondiert mit der Aufnahmehalterung 43 im Deckel 23 des Trommelmagazins 13. Bevorzugt kann das Halteelement 37 und/oder die Aufnahmehalterung 43 federnd ausgebildet sein.

Erfindungsgemäß hat das Stechelement 21 eine Kopplungsstruktur 39, in die das Kopplungselement 7 der Kopplungseinheit 6 eingreifen kann, um das Stechelement 21 anzukoppeln. Bevorzugt ist der Übergabeschenkel 31 des Stechelements 21 Bestandteil der Kopplungsstruktur 39. Die Kopplungsstruktur 39 kann beispielsweise als doppel-U-förmige Klammer 40 derart ausgebildet sein, dass das Kopplungselement 7 von der langen Schmalseite des Stechelements 21 ankoppeln kann. Eine Anschlagzunge 41 erstreckt sich quer zur Einstichrichtung in die Klammer 40 hinein, so dass das von der langen Schmalseite in die Klammer 40 eingeschobene Kopplungselement 7 bis an die Anschlagzunge 41 heran bewegt werden kann, wodurch seine Kopplungsposition bestimmt wird. Die Klammer 40 und die Anschlagzunge 41 sind so ausgebildet, dass sie das Kopplungselement 7 formschlüssig umgreifen können.

An der der Haltestruktur 39 gegenüberliegenden langen Schmalseite des Stechelements 21 sind zwei Führungsnasen 42 angeordnet, die mit der Führungsnuten 19d in der Kammern 16 korrespondieren, so dass das Stechelement 21 auf seinem Bewegungsweg durch die Führungsnasen 42 in den Führungsnuten 19d geführt werden kann.

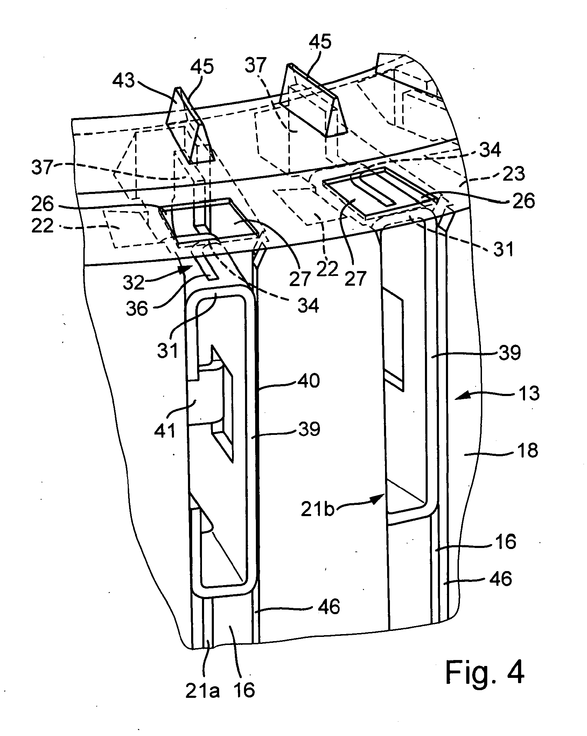

In Figur 4 ist das Trommelmagazin 13 mit Deckel 23 als perspektivische Detailansicht dargestellt. Die Stechelemente 21 werden derart in den Kammern 16 gehalten, dass die Kopplungsstrukturen 39 von der langen Schmalseite 19a zugänglich sind. Ein neues, unbenutztes Stechelement 21a (links in Figur 4) ist in seiner Ausgangsposition in den Kammern 19 derart gelagert, dass der Übergabeschenkel 31 vom Deckel 23 beabstandet und die Spitze 29 in der Kammer 16 enthalten ist. Die Kopplungsstruktur 39 schließt bündig mit der Außenseite des Trommelmagazins 13 ab, die von einer transparenten Folie 24 umschlossen ist. Das Stechelement 21a wird an seiner Kopplungsstruktur 39 kraftschlüssig von den Rändern 46 der langen Schmalseite 19a gehalten. Die Kopplungsstruktur 39 ist also in die lange Schmalseite 19a eingeklemmt. Alternativ kann auch eine Haltestruktur an der Kammer vorgesehen sein, in der das Stechelement kraftschlüssig gehalten wird.

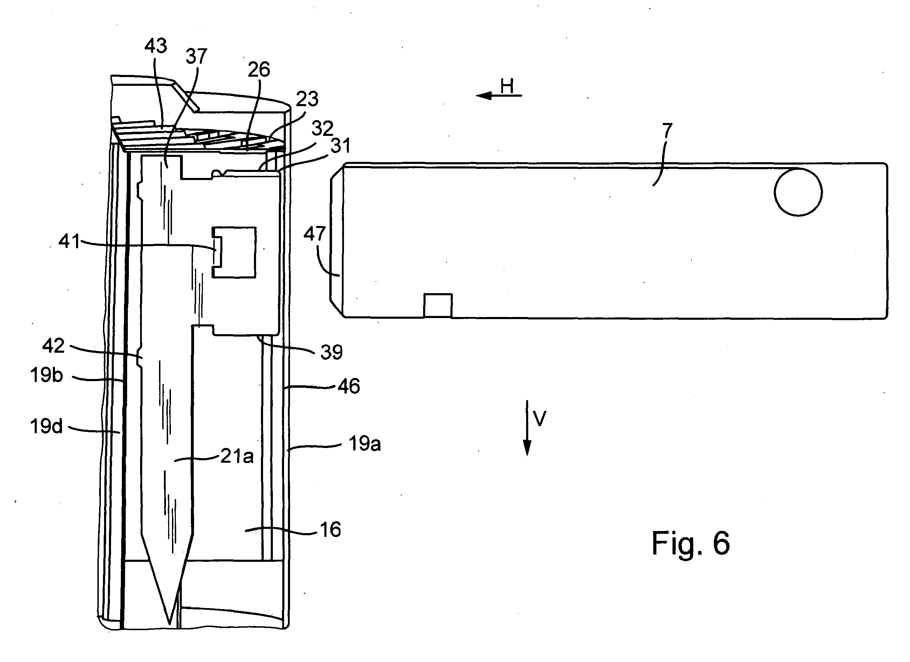

Figur 6 zeigt einen Schnitt durch die Kammer 16 des Trommelmagazins 13 mit einem unbenutzten Stechelement 21a, das in seiner Ausgangsposition angeordnet ist. Die gezeigte Kammer 16 mit dem Fingerkonus 12 fluchtet. Das Kopplungselement 7 ist als flacher Greifarm ausgebildet, der mit der Kopplungsstruktur 39 korrespondiert.

Zum Ankoppeln des Kopplungselements 7 an das Stechelement 21 wird das Kopplungselement 7 in Pfeilrichtung H bewegt, bis seine Vorderseite 47 an der Anschlagzunge 41 anschlägt. In einer bevorzugten Ausführungsform des Verfahrens zur Erzeugung einer Einstichwunde wird das Kopplungselement 7 weiter in Radialrichtung auf die Symmetrieachse des Trommelmagazins 18 zubewegt. Dabei wird das unbenutzte Stechelement 21 aus der Verklemmung zwischen den Rändern 46 der langen Schmalseite 19b gelöst und radial nach innen bewegt bis es eine Startposition erreicht. In der Startposition stoßen die Führungsnasen 42 an der Führungsnut 19d der Schmalseite 19b an, so dass das Stechelement 21 auf seinem Bewegungsweg geführt werden kann. Der Ankoppelvorgang des Kopplungselement 7 an das Stechelement 21 geht somit in den Bewegungsvorgang des Stechelements von der Ausgangsposition in die Startposition über.

Das kraftschlüssige Halten des Stechelements 21 in der Ausgangsposition weist den Vorteil auf, dass die unbenutzten Stechelemente 21 fixiert sind und ihre Spitzen 21 nicht aus der Austrittsöffnung 20c herausragen können. Gleichzeitig ermöglicht das kraftschlüssige Fixieren jedoch ein einfaches Ankoppeln und Lösen aus der Ausgangsposition durch das Kopplungselement 7.

Die um den Umfang des Trommelmagazins 13 gespannte Folie 24 wird während des Ankoppelns von dem Kopplungselement 7 durchstochen, so dass die Kammer 16, in der das anzukuppelnde Stechelement 21 enthalten ist, an der langen Schmalseite 19a geöffnet wird. Die anderen Kammern 16 bleiben jedoch verschlossen.

Zum Erzeugen einer Wunde wird das Kopplungselement 7 in Einstichrichtung bewegt, was in Figur 6 als Bewegung nach unten in Richtung des Pfeils V angedeutet ist. Dabei wird das Stechelement 21 soweit nach unten bewegt, bis seine Spitze 29 durch die Austrittsöffnung 20c aus der Kammer 16 hinausragt und sich in den Fingerkonus 12 erstreckt.

Nach Erreichen des Umkehrpunkts wird das Stechelement 21 von dem Kopplungselement 7 entgegen der Pfeilrichtung V bewegt, bis das Stechelement die Übergabeposition erreicht, so dass der Übergabeschenkel 31 mit der Probenübergabezone 32 zu dem Analyseelement 26 des Deckels 23 benachbart. Ein Transfer der in dem Kapillarkanal 34 gesammelten Flüssigkeit zur Probenkontaktzone 27 ist möglich.

Figur 4 zeigt ein Stechelement 21 b, das in der Übergabeposition positioniert ist. Die Probenübergabezone 32 und die Probenkontaktzone 27 sind bevorzugt aneinander angepasst. Sie können auch Flächen sein. Besonders bevorzugt hat die Probenübergabezone 32 eine Kapillarstruktur. Beispielsweise kann sie als Rinne ausgebildet sein oder einen gekrümmten oder gebogenen Querschnitt haben. Der Übergabeschenkel 31 ist am Deckel 23 derart angeordnet, das von der Probenübergabezone 32 (am Übergabeschenkel 31) Flüssigkeit auf die Probenkontaktzone 27 transferiert werden kann. Das Analyseelement 26 ist bevorzugt derart am Deckel angeordnet, dass die Probenkontaktzone 27 quer zur Einstichrichtung ausgerichtet ist. Die Flächennormale der Probenkontaktzone 27 weist dabei wenigstens eine in Einstichrichtung gerichtete Komponente auf, bevorzugt ist die Flächennormale in Einstichrichtung orientiert. Der Probentransfer von der Probenübergabezone 32 auf die Probenkontaktzone 27 findet daher ebenfalls in bzw. entgegen der Einstichrichtung statt. Dies hat zur Folge, dass das Stechelement 21 zum Erreichen der Übergabeposition vom Umkehrpunkt der Stechbewegung ausschließlich eine Bewegungskomponente entgegen der Einstichrichtung aufweist. Andere Bewegungen (nicht entgegen der Einstichrichtung) werden nicht ausgeführt. Das erfindungsgemäße Analysesystem wird hierdurch besonders einfach, zuverlässig und robust. Die (im Bild 4 nicht dargestellte) optische Messeinrichtung 9 erfasst die aufgrund der Reaktion in dem Reagenzsystem des Analyseelements 26 stattfindende Änderung der Messgröße. Die Mess- und Auswerteeinheit 8 bestimmt aus der Messgröße den gewünschten Analyten. Die optische Messeinrichtung 9 ist in Einstichrichtung hinter dem Analyseelement 26 angeordnet, also in axialer Richtung hinter dem Magazin 3. Dadurch lässt sich eine sehr schlanke Bauform des Analysegeräts realisieren.

Die Übergabeposition ist vorzugsweise auch gleichzeitig die Endposition, in der das Halteelement 37 des Stechelements 21 in die Klemmlasche 45 im Deckel 23 eingreift und kraftschlüssig gehalten wird.

Da in der Regel zur Übertragung der Körperflüssigkeit aus dem Kapillarkanal 34 des Stechelements 21 auf das Analyseelement 26 kein direkter Kontakt zwischen Analyseelement 26 und Stechelement 21 vorhanden sein muss, kann das Stechelement in seiner Endposition derart gehalten sein, dass der Übergabeschenkel 31 das Analyseelement 26 nicht berührt. Hierdurch wird eine Verformung und Beschädigung des Deckels vermieden, die dazu führen könnte, dass eine Positionierung bzw. ein Drehen des Trommelmagazins 13 um seine Drehachse gestört wird. Das Stechelement 21 ist derart gehalten, dass seine Spitze 29 in der Kammer 16 angeordnet ist.

Sobald sich das Stechelement 21 in seiner Endposition befindet, wird das Kopplungselement 7 radial nach außen bewegt und dabei aus der Kopplungsstruktur 39 herausgezogen. Da das Stechelement 21 von der Aufnahmehalterung 43 gehalten wird, bleibt es in seiner Endposition. Bevorzugt wird das Stechelement 21 in der Endposition remagaziniert, so dass das benutzte Element 21 in dem Magazin 3 verbleiben kann. Der Benutzer muss die gebrauchten Stechelemente 21 nicht einzeln entsorgen.

Das benutzte Stechelement 21 b nimmt in Figur 4 im Vergleich zu dem unbenutzten Stechelement 21 a eine andere Position in der Kammer ein. Hierdurch wird vorteilhafterweise verhindert, dass das Kopplungselement 7 an ein benutztes Stechelement 21 ankoppeln kann. Eine Mehrfachverwendung eines Stechelements 21 ist so ausgeschlossen. Beispielsweise kann durch entsprechende Ausbildung einer Kulissensteuerung der Kopplungseinheit 6 erzielt werden, dass das Kopplungselement 7 nur auf Höhe der Kopplungsstruktur 39 eines unbenutzten Stechelements 21 a in radialer Richtung auf die Drehachse des Trommelmagazins 13 zubewegt werden kann.

## Patentansprüche

1. Analysesystem zur Bestimmung eines Analyten in einer Körperflüssigkeit, umfassend
ein wiederverwendbares Analysegerät (2) und
ein Magazin (3) mit einer Mehrzahl von Stechelementen (21) und mit einer Mehrzahl von Analyseelementen (26), die eine Probenkontaktzone 27 und ein Reagenzsystem mit wenigstens einer eine Reagenz enthaltene Komponente umfassen, dessen Reaktion mit einer Körperflüssigkeit eine messbare Änderung einer Messgröße erzeugt,
wobei
das Analysegerät (2) umfasst
einen Antrieb (5), durch den die Stechbewegung des Stechelements (21) angetrieben wird, um es auf einem Bewegungsweg während einer Vortriebsphase in Einstichrichtung und nach Erreichen eines Umkehrpunktes der Stechbewegung während einer Rückführungsphase entgegen der Einstichrichtung zu bewegen,
eine Kopplungseinheit (6) mit einem Kopplungselement (7), die dazu ausgebildet ist, ein Stechelement (21) mit dem Antrieb (5) zu koppeln,
eine Mess- und Auswerteeinheit (8) zur Messung einer für die Bestimmung eines Analyten charakteristischen Messgröße und zur Ermittlung eines gewünschten Analyseergebnisses und
eine Halterung (10) zur Aufnahme des Magazins (3),
das Magazin (3) umfasst
eine Mehrzahl von nebeneinander angeordneten Kammern (16),
wobei
wenigstens eine Kammer (16) ein Stechelement (21) mit einer Spitze (29) enthält, das eine Kopplungsstruktur (39) aufweist, um das Stechelement (21) in einer Ausgangsposition an das Kopplungselement (7) der Kopplungseinheit (6) zu koppeln, und das auf dem Bewegungsweg der Stechbewegung in Einstichrichtung bewegbar ist,
die Kammern (16) in Einstichrichtung eine Austrittsöffnung (20c) aufweisen durch die ein auf dem Bewegungsweg bewegtes Stechelement (21) wenigstens teilweise aus der Kammer (16) heraustreten kann,
die Analyseelemente (26) stationär in Bezug auf das Magazin (3) angeordnet sind,
das Stechelement (21) eine Probenübergabezone (32) und einen Kapillarkanal (34) mit wenigstens einem Probeneinlass (35) und einem Probenauslass (36) hat,
der Bewegungsweg des Stechelements (21) eine Übergabeposition aufweist, in der das Stechelement (21) relativ zu dem Analyseelement (26) derart angeordnet ist, dass die Probenübergabezone (32) des Stechelements (21) der Probenkontaktzone (27) des Analyseelements (26) so benachbart ist, dass eine Übertragung einer Körperflüssigkeit aus dem Kapillarkanal (34) auf die Probenkontaktzone (27) stattfinden kann.

2. Analysesystem nach Anspruch 1, **dadurch gekennzeichnet, dass** das Magazin eine Mehrzahl von nebeneinander angeordneten langgestreckten Kammern (16) aufweist, die durch zwei in Längsrichtung der Kammern (16) verlaufende Seitenwände (17) begrenzt sind und deren Höhe und Länge größer ist als ihre durch den Abstand zweier benachbarter Seitenwände (17) definierte Breite, wobei die Kammern (16) von den Seitenwänden (17) begrenzte Hauptseiten sowie je zwei sich zwischen den Seitenwänden (14) erstreckende lange und kurze Schmalseiten (19a,19b,20a,20b) aufweisen, und
die Austrittsöffnung (20c) an der in Einstichrichtung vorderen kurzen Schmalseite (20a) angeordnet ist.

3. Analysesystem nach Anspruch 1 oder 2, **dadurch gekennzeichnet, dass** die Mess- und Auswerteeinheit (8) eine optische Messeinrichtung (9) umfasst, um eine optisch messbare, für die Bestimmung des Analyten charakteristische Messgröße zu messen.

4. Magazin für ein Analysegerät (2), vorzugsweise als Bestandteil eines Analysesystems nach Anspruch 1, mit einer Mehrzahl von Stechelementen (21) und mit einer Mehrzahl von Analyseelementen (26), die eine Probenkontaktzone (27) und ein Reagenzsystem mit wenigstens einer eine Reagenz enthaltene Komponente umfassen, dessen Reaktion mit einer Körperflüssigkeit eine messbare Änderung einer Messgröße erzeugt,
wobei
das Magazin (3) eine Mehrzahl von Kammern (16) hat,
wenigstens eine Kammer (16) ein Stechelement (21) mit einer Spitze (29) enthält, das eine Kopplungsstruktur (39) aufweist, um das Stechelement (21) in einer Ausgangsposition an ein Kopplungselement (7) einer Kopplungseinheit (6) des Analysegeräts (2) zu koppeln, und das auf dem Bewegungsweg der Stechbewegung in Einstichrichtung bewegbar ist,
die Kammern (16) eine Austrittsöffnung (20c) aufweisen,
durch die ein auf dem Bewegungsweg bewegtes Stechelement (21) wenigstens teilweise aus der Kammer (16) heraustreten kann, und
die Analyseelemente stationär in Bezug auf das Magazin (3) angeordnet sind,
das Stechelement (21) eine Probenübergabezone (32) und einen Kapillarkanal (34) mit wenigstens einem Probeneinlass (35) und einem Probenauslass (36) hat,
der Bewegungsweg des Stechelements (21) eine Übergabeposition aufweist, in der das Stechelement (21) relativ zu dem Analyseelement (26) derart angeordnet ist, dass die Probenübergabezone (32) des Stechelements (21) der Probenkontaktzone (27) des Analyseelements (26) so benachbart ist, dass eine Übertragung einer Körperflüssigkeit aus dem Kapillarkanal (34) auf die Probenkontaktzone (27) stattfindet.

5. Magazin nach Anspruch 4, **dadurch gekennzeichnet, dass**
das Magazin (3) eine Mehrzahl von benachbart angeordneten langgestreckten Kammern (16) aufweist,
die Kammern (16) durch zwei in Längsrichtung der Kammern (16) verlaufende Seitenwände (17) begrenzt sind, deren Höhe und Länge größer ist als der Abstand zweier benachbarter Seitenwände (14),
die Kammer (16) von den Seitenwänden (17) begrenzte Hauptseiten sowie je zwei sich zwischen den Seitenwänden (17) erstreckende lange und kurze Schmalseiten (19a,19b,20a,20b) aufweisen,
die Kammern (16) an der in Einstichrichtung vorderen kurzen Schmalseite (20a) die Austrittsöffnung (20c) aufweisen.

6. Analysesystem oder Magazin nach einem der vorhergehenden Ansprüche, **dadurch gekennzeichnet, dass** die Kammern (16) an der in Einstichrichtung hinteren kurzen Schmalseite (20b) eine Analyseöffnung (20d) aufweisen, die den Austrittsöffnungen (20c) gegenüberliegen, und das Magazin (3) einen Deckel (23) aufweist, der die Analyseöffnungen (20d) der Kammern (16) an der hinteren kurzen Schmalseite (20b) verschließt.

7. Analysesystem oder Magazin nach Anspruch 6, **dadurch gekennzeichnet, dass**
das Analyseelement (26) derart an dem Deckel (23) des Magazins (3) angeordnet ist, dass die Probenkontaktzone (27) des Analyseelements (26) mit der Analyseöffnung (20d) der Kammer (16) fluchtet,
die Probenübergabezone (32) am hinteren Ende (33) des Stechelements (21) angeordnet ist,
die Probenübergabezone (32) und die Probenkontaktzone (27) aneinander angepasst sind.

8. Analysesystem oder Magazin nach Anspruch 7, **dadurch gekennzeichnet, dass**
das Stechelement (21) zwei Schenkel aufweist,
wobei
der eine Schenkel ein Übergabeschenkel (31) ist, der sich quer zur Einstichrichtung erstreckt und an dem die Probenübergabezone (32) angeordnet ist, und
der andere Schenkel ein Stechschenkel (28) ist, der die Spitze (29) des Stechelements (21) umfasst und sich in Einstichrichtung erstreckt,
der Übergabeschenkel (31) in der Übergabeposition zu dem Deckel (23) derart benachbart ist, dass eine Übertragung einer Körperflüssigkeit von der Probenübergabezone (32) an die Probenkontaktzone (27) stattfindet.

9. Analysesystem oder Magazin nach Anspruch 8, **dadurch gekennzeichnet, dass** der Übergabeschenkel (31) des Stechelements (21) Bestandteil der Kopplungsstruktur ist.

10. Analysesystem oder Magazin nach einem der vorhergehenden Ansprüche, **dadurch gekennzeichnet, dass** das Magazin (3) ein Trommelmagazin (13) ist und sich die Kammern (16) in axialer Richtung der Magazintrommel (18) erstrecken.

11. Analysesystem oder Magazin nach einem der vorhergehenden Ansprüche, **dadurch gekennzeichnet, dass** die Kammern (16) des Magazins (3) an einer ihrer langen Schmalseiten (19a) für das Kopplungselement (7) derart zugänglich sind, dass das Kopplungselement (7) an die Kopplungsstruktur (39) des Stechelements (21) formschlüssig ankoppelbar ist, wenn das Magazin (3) mit einem in der Kammer (16) positionierten Stechelement (21) in der Halterung (10) angeordnet ist.

12. Analysesystem oder Magazin nach Anspruch 11, **dadurch gekennzeichnet, dass** die Kammern (16) des Magazins (3) an der langen Schmalseite (19b), an der die Kammern (16) zugänglich sind, und/oder an der kurzen Schmalseite (20a), an der sich die Austrittsöffnung (20c) der Kammern (16) befindet, mit einer Folie (24,25) verschlossen sind.

13. Analysesystem oder Magazin nach Anspruch 11, **dadurch gekennzeichnet, dass** die Folie (24,25) mittels des Kopplungselements (7) der Kopplungseinheit (6) und/oder mittels des Stechelements (21) durchstoßbar ist.

14. Analysesystem oder Magazin nach Anspruch 6, **dadurch gekennzeichnet, dass** der Deckel (23) eine Aufnahmehalterung (43) umfasst, in der das Stechelement (21) nach dem Einstich kraftschlüssig in einer Endposition gehalten wird, in der sich die Spitze (29) des Stechelements (21) in der Kammer (16) befindet.

15. Analysesystem oder Magazin nach einem der vorhergehenden Ansprüche, **dadurch gekennzeichnet, dass** die Kammer (16) des Magazins (3) eine Haltestruktur umfasst, in der das Stechelement (21) in einer Ausgangsposition vor dem Stechvorgang kraftschlüssig gehalten wird, wobei das Stechelement (21) bevorzugt mittels des Kopplungselementes (7) der Kopplungseinheit (6) aus seiner Ausgangsposition quer zur Einstichrichtung herausbewegbar ist.

16. Magazin mit einem Analyseelement, insbesondere als Bestandteil eines Analysesystems nach Anspruch 1, wobei
- ein Stechelement (21) in dem Magazin (3) enthalten ist,
- das Stechelement (21) eine Probenübergabezone (32) und einen Kapillarkanal (34) mit wenigstens einem Probeneinlass (35) und einem Probenauslass (36) hat,
- das Stechelement (21) eine Kopplungsstruktur (39) aufweist, an die ein Kopplungselement (7) einer Kopplungseinheit (6) ankoppelbar ist,
- das Stechelement (21) zwei Schenkel aufweist,
- der eine Schenkel ein Übergabeschenkel (31) ist, der sich quer zur Einstichrichtung erstreckt und an dem die Probenübergabezone (32) angeordnet ist,
- der andere Schenkel ein sich in Einstichrichtung erstreckender Stechschenkel (28) ist, der eine Spitze (29) des Stechelements (21) und den Probeneinlass (35) des Kapillarkanals (34) umfasst und
- das Stechelement (21) derart angeordnet ist, dass der Übergabeschenkel (31) dem in dem Magazin (3) fixierten Analyseelement (26) gegenüber angeordnet ist.

17. Magazin nach Anspruch 16, **dadurch gekennzeichnet, dass** der Übergabeschenkel (31) des Stechelements (21) Bestandteil der Kopplungsstruktur (39) ist.

18. Analysesystem nach einem der vorhergehenden Ansprüche, **dadurch gekennzeichnet, dass** an dem in dem Magazin fixierten Analyseelement (26) die Messung der charakteristischen Messgröße und die Ermittlung des gewünschten Analyseergebnisses mittels der Mess- und Auswerteeinheit (8) erfolgt, wenn das Stechelement (21) (am Ende seines Bewegungsweges) nach Erreichen des Umkehrpunktes der Stechbewegung wieder in der Kammer (16) angeordnet ist, bevorzugt, wenn das Stechelement (21) remagaziniert ist.

19. Verfahren zum Erzeugen einer Wunde in einem Körperteil und zur Untersuchung einer aus der Wunde austretenden Körperflüssigkeit
mittels eines Analysesystems, welches ein wiederverwendbares Analysegerät (2) und ein Magazin (3) mit einer Mehrzahl von Stechelementen (21) mit einer Spitze (29) und mit einer Mehrzahl von Analyseelementen (26) hat,
wobei
das Analysegerät (1) einen Antrieb (4), eine Kopplungseinheit (6) mit einem Kopplungselement (7), um das Stechelement (21) mit dem Antrieb (4) zu koppeln, eine Mess- und Auswerteeinheit (8) zur Messung einer für die Bestimmung eines Analyten charakteristischen Messgröße und zur Ermittlung eines gewünschten Analyseergebnisses und eine Halterung (10) zur Aufnahme des Magazins (3), umfasst,
das Magazin (3) eine Mehrzahl von nebeneinander angeordneten langgestreckten Kammern (16) umfasst
die Kammern (16) je ein Stechelement (21) enthalten, das eine Kopplungsstruktur (39) hat, und die Kammern (16) derart zugänglich sind, dass das Kopplungselement (7) der Kopplungseinheit (6) des Analysegeräts (2) formschlüssig an die Kopplungsstruktur (39) des Stechelements (21) ankoppelbar ist, und
das Stechelement (21) eine Probenübergabezone (32) und einen Kapillarkanal (34) mit wenigstens einem Probeneinlass (35) und einem Probenauslass (36) hat,
die Analyseelemente (26) eine Probenkontaktzone (27) und ein Reagenzsystem mit wenigstens einer eine Reagenz enthaltene Komponente umfassen, dessen Reaktion mit einer Körperflüssigkeit eine messbare Änderung einer Messgröße erzeugt,
die Analyseelemente (26) stationär in Bezug auf das Magazin (3) angeordnet sind,
insbesondere mittels eines Analysesystems (1) nach Anspruch 1,
mit folgenden Schritten:
a) Formschlüssiges Ankoppeln des Kopplungselements (7) an die Kopplungsstruktur (39) des in einer Ausgangsposition positionierten Stechelements (21),
b) Bewegen des Stechelements (21) auf einem Bewegungsweg während einer Vortriebsphase in Einstichrichtung bis zum Erreichen eines Umkehrpunktes der Stechbewegung,
c) Erzeugen eine Einstichwunde in der Haut eines Körperteils,
d) Übertragen einer Körperflüssigkeit durch den Probeneinlass (35) in den Kapillarkanal (34),
e) Bewegen des Stechelements (21) entgegen der Einstichrichtung in eine Übergabeposition, in der das Stechelement (21) derart in der Kammer (16) des Magazins (3) positioniert ist, dass die Probenübergabezone (32) des Stechelements (21) zu der Probenkontaktzone (27) des Analyseelements (26) benachbart ist und eine Übertragung der Körperflüssigkeit aus dem Kapillarkanal (34) auf die Probenkontaktzone (27) stattfindet,
f) Messen einer Messgröße, die für die Bestimmung eines Analyten charakteristisch ist, an der Probenkontaktzone (27).

20. Verfahren nach Anspruch 19, wobei
die Kammern (16) des Magazins (3) an der in Einstichrichtung hinteren kurzen Schmalseite (20b) Analyseöffnungen (20d) aufweisen, die den Austrittsöffnungen (20c) gegenüberliegen,
das Magazin (3) einen Deckel (23) hat, der die Analyseöffnungen (20d) der Kammern (16) verschließt,
das Analyseelement (26) derart am Deckel (23) des Magazins (3) angeordnet ist, dass die Probenkontaktzone (27) des Analyseelements (26) mit der Analyseöffnung (20d) der Kammer (16) fluchtet,
das Stechelement (21) zwei Schenkel aufweist, der eine Schenkel ein Übergabeschenkel (31) ist, der sich quer zur Einstichrichtung erstreckt und an dem die Probenübergabezone (32) angeordnet ist, und der andere Schenkel ein Stechschenkel (28) ist, der die Spitze (29) des Stechelements (21) umfasst und sich in Einstichrichtung erstreckt
**gekennzeichnet durch** den folgenden Schritt:
Bewegen des Stechelements (21) entgegen der Einstichrichtung bis der Übergabeschenkel (32) des Stechelements (21) am Deckel (23) des Magazins anliegt.

21. Verfahren nach Anspruch 20, wobei
der Deckel eine Aufnahmehalterung (43) umfasst und
das Stechelement (21) ein Halteelement (37) aufweist, das mit der Aufnahmehalterung (43) des Deckels (23) korrespondiert,
**gekennzeichnet durch** folgenden weiteren Schritt:
g) Bewegen des Stechelements (21) in eine Endposition, in der das Halteelement (37) des Stechelements (21) in der Aufnahmehalterung (43) des Deckels (23) derart gehalten wird, dass die Spitze (29) des Stechelements (21) in der Kammer (16) positioniert ist.

22. Verfahren nach einem der Ansprüche 19 bis 21, **gekennzeichnet durch** folgenden weiteren Schritt:
Bewegen des Stechelements (21) quer zur Einstichrichtung aus einer Ausgangsposition heraus, in der das unbenutzte Stechelement (21) vor dem Gebrauch gelagert ist, in eine Startposition, in der das Stechelement (21) mit einer langen Schmalseite (19b) der Kammer (16) so in Kontakt steht, dass es auf dem Bewegungsweg der Stechbewegung von der langen Schmalseite (19b) geführt wird.
